# EUROPEAN PATENT APPLICATION

(11) **EP 4 640 679 A1**
(43) Date of publication of application: **29.10.2025**
(21) Application number: 23906041.1
(22) Date of filing: 21.12.2023
(51) Int. Cl.: C07D 401/12, C07D 401/14, C07D 409/14, C07D 417/14, A61K 31/444, A61K 31/497, A61P 29/00, A61P 37/00

(54) **SALT FORM AND CRYSTAL FORM OF PYRIDINE MULTI-SUBSTITUTED COMPOUND AND PREPARATION METHOD THEREFOR**

(30) Priority: 22.12.2022 CN 202211740788; 08.12.2023 CN 202311689614
(71) Applicant: Usynova Pharmaceuticals Ltd., Shanghai 201203 (CN)
(72) Inventor: QIAN, Wenyuan, Shanghai 200131 (CN); XU, Guanghai, Shanghai 200131 (CN)
(74) Representative: Cabinet Becker et Associés
(86) International application number: PCT/CN2023/140597
(87) International publication number: WO 2024/131882

(57) **Abstract**

Disclosed in the present invention are a salt form and a crystal form of a pyridine multi-substituted compound and a preparation method therefor. Specifically disclosed is the use of a crystal form of a compound of formula (I) and a preparation method therefor in the preparation of a drug for treating related diseases.

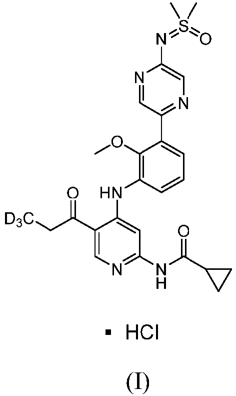

## Description

**This application claims the priority of:**
Application number: CN202211740788.0; Application date: December 22, 2022;
Application number: CN202311689614.0; Application date: December 8, 2023.

### TECHNICAL FIELD

The present disclosure relates to a salt form and a crystal form of a multi-substituted pyridine compound and a preparation method thereof, in particular to a crystal form of a compound of formula (I) and a preparation method thereof use thereof in the manufacuture of a medicament for treating related diseases.

### BACKGROUND

The family of Janus kinases (JAKs) of non-receptor tyrosine kinases is critical in mediating the signaling of numerous cytokines that cause inflammation. The JAK family includes JAK1, JAK2, JAK3, and TYK2. TYK2 and JAK1/2/3 usually work in pairs, or as "dimers," to transmit signals from extracellular cytokines to the cell nucleus. TYK2 selectively participates in the transmission of proinflammatory cytokines such as IL-23, IL-12, and type I IFN signals. Therefore, TYK2 inhibitors can be an effective treatment for a variety of severe inflammatory and autoimmune diseases.

JAK1, JAK2, JAK3 and TYK2 in the JAK protein family all have JAK homology domains (JH), of which the JH1 domain is also called the kinase domain and JH2 is a pseudokinase domain. Although the JH2 domain of TYK2 is very similar to the JH1 domain and contains an ATP binding site that is very similar to the JH1 domain, specific residue differences exclude the catalytic function of JH2. Although the specific mechanism by which TYK2 achieves allosteric inhibition through the JH2 domain has not been elucidated, there is evidence that small molecule ligands that bind to JH2 stabilize the autoinhibitory interaction between the JH2 domain and the JH1 active site. These interactions between JH2 and JH1 are believed to limit the conformational mobility of the JH1 active site required for phosphorus transfer catalysis. Inhibiting TYK2 by binding to the JH2 domain of TYK2 can maintain a high level of selectivity for other JAK family members and the entire kinase family.

Current TYK2 inhibitors mainly include orthosteric inhibitors that inhibit the kinase domain (JH1) and allosteric inhibitors that inhibit the pseudokinase domain (JH2). Orthosteric inhibitors are represented by Pfizer's PF-06826647, which is used to treat diseases such as plaque and ulcerative colitis, and is currently in phase II clinical trials. Allosteric inhibitors are represented by BMS-986165. The clinical trial of BMS-986165 for the treatment of massive psoriasis has been advanced to Phase III, with outstanding clinical effects and good safety. At the same time, BMS-986165 is also in clinical research for a variety of autoimmune diseases including Crohn's disease, psoriatic arthritis, and systemic lupus erythematosus. In addition to BMS-986165, Nimbus also has several TYK2 allosteric inhibitors in preclinical screening, and Fronthera's TYK2 allosteric inhibitor FTP-637 that has been acquired by Hisco (reported recently) is preparing to enter phase I clinical trials.

### SUMMARY

The present disclosure provides a compound of formula (I),

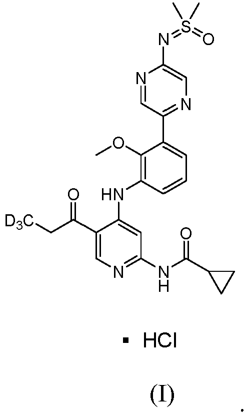

The present disclosure also provides a crystal form A of the compound of formula (I), characterized in that the X-ray powder diffraction pattern has characteristic diffraction peaks at the following 2θ angles: 17.56±0.20°, 24.36±0.20°, 25.69±0.20°, and 27.37±0.20°.

In some embodiments of the present disclosure, the X-ray powder diffraction pattern of the crystal form A of the above-mentioned compound (I) has characteristic diffraction peaks at the following 2θ angles: 8.70±0.20°, 10.05±0.20°, 12.08±0.20°, and 17.56±0.20°.

In some embodiments of the present disclosure, the crystal form A of the compound of formula (I) is characterized in that the X-ray powder diffraction pattern of the crystal form A at least contains 6, 7 or 8 diffraction peaks expressed by 2θ angle selected from: 17.56±0.20°, 18.76±0.20°, 19.63±0.20°, 20.44±0.20°, 22.89±0.20°, 24.36±0.20°, 25.69±0.20°, and 27.37±0.20°.

In some embodiments of the present disclosure, the crystal form A of the compound of formula (I) is characterized in that the X-ray powder diffraction pattern of the crystal form A at least contains 6, 7 or 8 diffraction peaks expressed by 2θ angle selected from: 8.70±0.20°, 10.05±0.20°, 12.08±0.20°, 15.56±0.20°, 17.56±0.20°, 18.76±0.20°, 19.35±0.20°, and 20.44±0.20°.

In some embodiments of the present disclosure, the X-ray powder diffraction pattern of the crystal form A of the above-mentioned compound (I) has characteristic diffraction peaks at the following 2θ angles: 17.56±0.20°, 18.76±0.20°, 19.63±0.20°, 20.44±0.20°, 22.89±0.20°, 24.36±0.20°, 25.69±0.20°, and 27.37±0.20°.

In some embodiments of the present disclosure, the X-ray powder diffraction pattern of the crystal form A of the above-mentioned compound (I) has characteristic diffraction peaks at the following 2θ angles: 8.70±0.20°, 10.05±0.20°, 12.08±0.20°, 15.56±0.20°, 17.56±0.20°, 18.76±0.20°, 19.35±0.20°, and 20.44±0.20°.

Some of the crystal form A of the above-mentioned compound of formula (I) of the present disclosure are characterized in that the X-ray powder diffraction pattern of the crystal form A at least contains 10, 11 or 12 diffraction peaks expressed by 2θ angle selected from: 8.70±0.20°, 10.05±0.20°, 15.56±0.20°, 17.56±0.20°, 18.76±0.20°, 19.63±0.20°, 20.44±0.20°, 22.89±0.20°, 24.36±0.20°, 25.69±0.20°, 27.37±0.20°, and 29.49±0.20°.

Some of the crystal form A of the above-mentioned compound of formula (I) of the present disclosure are characterized in that the X-ray powder diffraction pattern of the crystal form A at least contains 10, 11 or 12 diffraction peaks expressed by 2θ angle selected from: 8.70±0.20°, 10.05±0.20°, 12.08±0.20°, 15.56±0.20°, 17.56±0.20°, 18.76±0.20°, 19.35±0.20°, 20.44±0.20°, 22.53±0.20°, 24.51±0.20°, 25.69±0.20°, and 27.37±0.20°.

In some embodiments of the present disclosure, the X-ray powder diffraction pattern of the crystal form A of the above-mentioned compound (I) has characteristic diffraction peaks at the following 2θ angles: 8.70±0.20°, 10.05±0.20°, 12.08±0.20°, 15.56±0.20°, 17.56±0.20°, 19.63±0.20°, 20.44±0.20°, 22.89±0.20°, 24.36±0.20°, 25.69±0.20°, 27.37±0.20°, and 29.49±0.20°.

In some embodiments of the present disclosure, the X-ray powder diffraction pattern of the crystal form A of the above-mentioned compound (I) has characteristic diffraction peaks at the following 2θ angles: 8.70±0.20°, 10.05±0.20°, 12.08±0.20°, 15.56±0.20°, 17.56±0.20°, 18.76±0.20°, 19.35±0.20°, 20.44±0.20°, 22.53±0.20°, 24.51±0.20°, 25.69±0.20°, and 27.37±0.20°.

In some embodiments of the present disclosure, the crystal form A of the compound of formula (I) is characterized in that the X-ray powder diffraction pattern of the crystal form A at least contains 12, 13, 14, 15 or 16 diffraction peaks expressed by 2θ angle selected from: 8.70±0.20°, 10.05±0.20°, 12.08±0.20°, 15.56±0.20°, 17.56±0.20°, 18.76±0.20°, 19.63±0.20°, 20.44±0.20°, 22.89±0.20°, 23.77±0.20°, 24.36±0.20°, 25.69±0.20°, 26.33±0.20°, 27.37±0.20°, 27.83±0.20°, and 29.49±0.20°.

In some embodiments of the present disclosure, the crystal form A of the compound of formula (I) is characterized in that the X-ray powder diffraction pattern of the crystal form A at least contains 12, 13, 14, 15 or 16 diffraction peaks expressed by 2θ angle selected from: 8.70±0.20°, 10.05±0.20°, 12.08±0.20°, 14.63±0.20°, 15.56±0.20°, 17.56±0.20°, 18.76±0.20°, 19.35±0.10°, 19.63±0.10°, 20.44±0.20°, 22.53±0.20°, 22.89±0.10°, 24.51±0.20°, 25.69±0.20°, 26.33±0.20°, and 27.37±0.20°.

In some embodiments of the present disclosure, the X-ray powder diffraction pattern of the crystal form A of the above-mentioned compound (I) has characteristic diffraction peaks at the following 2θ angles: 8.70±0.20°, 10.05±0.20°, 12.08±0.20°, 14.63±0.20°, 15.56±0.20°, 16.39±0.20°, 17.56±0.20°, 19.63±0.20°, 20.44±0.20°, 22.89±0.20°, 23.77±0.20°, 24.36±0.20°, 25.69±0.20°, 26.33±0.20°, 27.37±0.20°, and 29.49±0.20°.

In some embodiments of the present disclosure, the X-ray powder diffraction pattern of the crystal form A of the above-mentioned compound (I) has characteristic diffraction peaks at the following 2θ angles: 8.70±0.20°, 10.05±0.20°, 12.08±0.20°, 14.63±0.20°, 15.56±0.20°, 17.56±0.20°, 18.76±0.20°, 19.35±0.10°, 19.63±0.10°, 20.44±0.20°, 22.53±0.20°, 22.89±0.10°, 24.51±0.20°, 25.69±0.20°, 26.33±0.20°, and 27.37±0.20°.

The present disclosure provides a crystal form A of a compound of formula (I), wherein the X-ray powder diffraction pattern has characteristic diffraction peaks at the following 2θ angles: 17.56°±0.20°, 24.36°±0.20°, 27.37°±0.20°, and/or 8.70°±0.20°, and/or 10.05°±0.20°, and/or 12.08°±0.20°, and/or 14.63°±0.20°, and/or 15.56°±0.20°, and/or 16.39°±0.20°, and/or 18.76°±0.20°, and/or 19.35°±0.20°, and/or 19.63°±0.20°, and/or 20.44°±0.20°, and/or 21.94°±0.20°, and/or 22.53°±0.20°, and/or 22.89°±0.20°, and/or 23.23°±0.20°, and/or 23.77°±0.20°, and/or 24.51°±0.20°, and/or 25.69°±0.20°, and/or 26.33°±0.20°, and/or 26.73°±0.20°, and/or 27.83°±0.20°, and/or 28.58°±0.20°, and/or 29.49°±0.20°, and/or 30.42°±0.20°, and/or 31.13°±0.20°, and/or 32.29°±0.20°, and/or 32.88°±0.20°, and/or 34.04°±0.20°, and/or 35.19°±0.20°, and/or 36.30°±0.20°, and/or 38.89°±0.20°.

The present disclosure provides a crystal form A of the compound of formula (I), wherein the X-ray powder diffraction pattern has characteristic diffraction peaks at the following 2θ angles: 17.56°, 24.36°, 27.37°, 8.70°, 10.05°, 12.08°, 14.63°, 15.56°, 16.39°, 18.76°, 19.35°, 19.63°, 20.44°, 21.94°, 22.53°, 22.89°, 23.23°, 23.77°, 24.51°, 25.69°, 26.33°, 26.73°, 27.83°, 28.58°, 29.49°, 30.42°, 31.13°, 32.29°, 32.88°, 34.04°, 35.19°, 36.30°, and 38.89°.

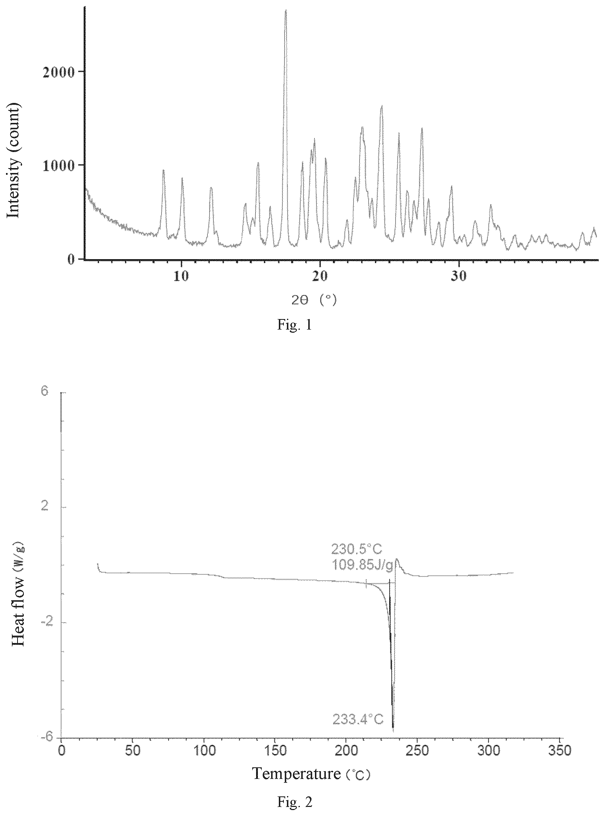

In some embodiments of the present disclosure, disclosed is the crystal form A of the above compound of formula (I), 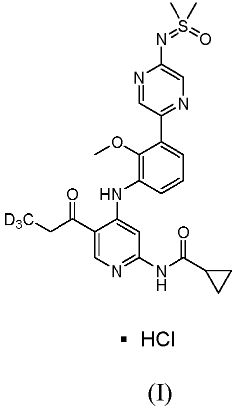 wherein the XRPD pattern is shown in Fig. 1.

In some embodiments of the present disclosure, the XRPD pattern analysis data of the above-mentioned crystal form A is shown in Table 1:

**Table 1: XRPD pattern analysis data of the crystal form of the compound of formula (I)**

| No. | 2θ angle (°) | Interplanar spacing (Å) | Height (cts) | Relative strength (%) | No. | 2θ angle (°) | Interplanar spacing (Å) | Height (cts) | Relative strength (%) |
|---|---|---|---|---|---|---|---|---|---|
| 1 | 8.70 | 10.16 | 726.25 | 30.86 | 18 | 24.51 | 3.63 | 1347.21 | 57.25 |
| 2 | 10.05 | 8.80 | 686.53 | 29.17 | 19 | 25.69 | 3.47 | 1178.34 | 50.07 |
| 3 | 12.08 | 7.33 | 592.63 | 25.18 | 20 | 26.33 | 3.39 | 561.95 | 23.88 |
| 4 | 14.63 | 6.06 | 465.10 | 19.76 | 21 | 26.73 | 3.34 | 451.09 | 19.17 |
| 5 | 15.56 | 5.69 | 835.74 | 35.51 | 22 | 27.37 | 3.26 | 1215.63 | 51.66 |
| 6 | 16.39 | 5.41 | 441.58 | 18.76 | 23 | 27.83 | 3.21 | 489.50 | 20.80 |
| 7 | 17.56 | 5.05 | 2353.25 | 100.00 | 24 | 28.58 | 3.12 | 256.54 | 10.90 |
| 8 | 18.76 | 4.73 | 838.14 | 35.62 | 25 | 29.49 | 3.03 | 631.42 | 26.83 |
| 9 | 19.35 | 4.59 | 1014.95 | 43.13 | 26 | 30.42 | 2.94 | 117.31 | 4.98 |
| 10 | 19.63 | 4.52 | 1117.46 | 47.49 | 27 | 31.13 | 2.87 | 284.48 | 12.09 |
| 11 | 20.44 | 4.34 | 906.02 | 38.50 | 28 | 32.29 | 2.77 | 470.90 | 20.01 |
| 12 | 21.94 | 4.05 | 292.55 | 12.43 | 29 | 32.88 | 2.72 | 235.52 | 10.01 |
| 13 | 22.53 | 3.95 | 733.88 | 31.19 | 30 | 34.04 | 2.63 | 153.91 | 6.54 |
| 14 | 22.89 | 3.89 | 1137.67 | 48.34 | 31 | 35.19 | 2.55 | 115.42 | 4.90 |
| 15 | 23.23 | 3.83 | 1093.27 | 46.46 | 32 | 36.30 | 2.48 | 142.46 | 6.05 |
| 16 | 23.77 | 3.74 | 504.26 | 21.43 | 33 | 38.89 | 2.32 | 103.53 | 4.40 |
| 17 | 24.36 | 3.65 | 1468.99 | 62.42 | | | | | |

In some embodiments of the present disclosure, the differential scanning calorimetry curve of the above-mentioned crystal form A has a starting point of an endothermic peak at 230.5°C±3.0°C.

In some embodiments of the present disclosure, the DSC spectrum of the above-mentioned crystal form A is shown in Fig. 2.

In some embodiments of the present disclosure, the thermogravimetric analysis curve of the above-mentioned crystal form A shows a weight loss of up to 1.02%±0.20% at 175.0°C±3.0°C.

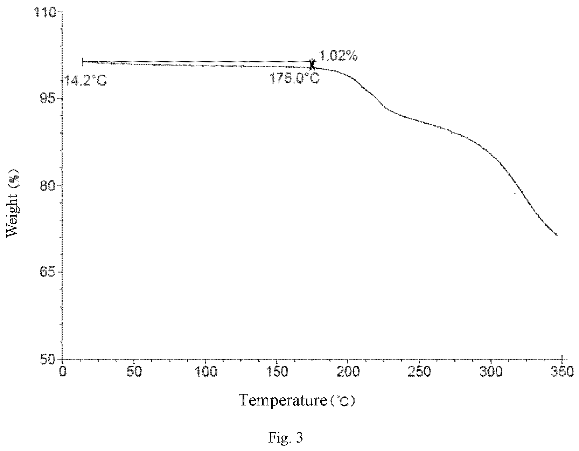

In some embodiments of the present disclosure, the TGA spectrum of the above-mentioned crystal form A is shown in Fig. 3.

The present disclosure also provides the following biological testing method:

### Pharmacokinetic evaluation of compounds in mice

Experimental purpose: To test the pharmacokinetics of compounds in Balb/c mice
Experimental materials: Male Balb/c mice, fasted
Experimental operation:

The pharmacokinetic characteristics of compound 1 in rodents after intravenous and oral administration were tested using standard protocols.

After the Balb/c mice arrived at the facility, they were adapted/quarantined for at least 3 days. After the adaptation/quarantine, the veterinarian or designated personnel checked the health status of the Balb/c mice to assess whether the animals are suitable for experimental research. All Balb/c mice were fasted overnight before dosing and resumed feeding 4 hours after dosing. In the experiment, the candidate compound was prepared into a homogenous solution and administered to Balb/c mice as a single intravenous injection and oral administration. The intravenous injection solvent was a clear solution of 80% polyethylene glycol 400/20% water, and the oral solvent was a homogenous suspension of ethanol/vitamin E polyethylene glycol succinate/polyethylene glycol 300 (5/5/90). The animals were weighed before dosing and the dosing volume was calculated based on the body weight. Whole blood samples were collected within 24 hours by jugular vein puncture, and all blood samples were immediately transferred to labeled commercial centrifuge tubes containing K2-EDTA. After the blood sample was collected, the blood sample was centrifuged at 3200g for 10 minutes at 4 °C. The obtained supernatant plasma was sucked and quickly placed in dry ice and then stored at -60°C or lower for LC-MS/MS analysis. The non-compartmental model was used to analyze the plasma drug concentration-time data and calculate the pharmacokinetic parameters by the WinNonlin software package (Version 6.3 and above). The PK parameters included (if data allowed) but were not limited to peak concentration (Cmax), time to peak (Tmax), elimination half-life (T1/2), area under the plasma concentration-time curve (AUC), mean residence time (MRT), bioavailability, etc.

### Technical Effects

The compound of the present disclosure has excellent pharmacokinetic properties and the crystal form of the compound of the present disclosure has good stability, and is suitable for drug development.

### Definition and Description

Unless otherwise specified, the following terms and phrases used herein are intended to have the following meanings. A particular phrase or term should not be considered to be uncertain or unclear in the absence of a special definition, but should be understood according to its ordinary meaning. When a trade name appears herein, it is intended to refer to its corresponding commercial product or its active ingredient.

The intermediate compounds of the present disclosure can be prepared by a variety of synthetic methods well known to those skilled in the art, including the specific embodiments listed below, the embodiments formed by combining them with other chemical synthesis methods, and equivalent alternatives well known to those skilled in the art. The alternative embodiments include but are not limited to the examples of the present disclosure.

The chemical reactions of the specific embodiments of the present disclosure are carried out in a suitable solvent, which must be suitable for the chemical changes of the present disclosure and the reagents and materials required. In order to obtain the compounds of the present disclosure, it is sometimes necessary for those skilled in the art to modify or select the synthesis steps or reaction processes based on the existing embodiments.

For any given crystal form, the relative intensity of the diffraction peaks can change due to the preferable orientation caused by factors such as crystal morphology, which is well known in the field of crystallography. Where there is a preferable orientation effect, the peak intensity changes, but the diffraction peak position of the crystal form cannot be changed. In addition, for any given crystal form, there may be a slight error in the position of the peak, which is also well known in the field of crystallography. For example, due to changes in temperature when analyzing the sample, the movement of the sample, or the calibration of the instrument, the position of the peak can move, and the measurement error of the 2θ value is sometimes about ±0.20° or ±0.10°. Therefore, it is well known to those skilled in the art that this error should be taken into account when determining each crystalline structure.

Unless otherwise stated, X-ray powder diffraction (XRPD) can detect information such as changes in crystal forms, crystallinity, and crystal structure states, and is a common means of identifying crystal forms. The peak position of the XRPD spectrum depends mainly on the structure of the crystal form, is relatively insensitive to experimental details, while its relative peak height depends on many factors related to sample preparation and instrument geometry. Therefore, in some embodiments, the crystal form of the present disclosure is characterized by an XRPD pattern with certain peak positions, which is substantially as shown in the XRPD pattern provided in the drawings of the present disclosure. At the same time, the measurement of 2θ of the XRPD spectrum may have experimental errors, and the measurement of 2θ of the XRPD spectrum may be slightly different between different instruments and different samples, so the value of 2θ cannot be regarded as absolute. According to the instrument conditions used in this experiment, the diffraction peak has an error tolerance of ±0.20° or ±0.10°.

DSC measures the transition temperature when a crystal absorbs or releases heat due to changes in its crystalline structure or melting of the crystal. For the same type of crystal form of the same compound, the error of thermal transition temperature and melting point is typically within about 5°C or 3°C in consecutive analyses. When we say that a compound has a given DSC peak or melting point, this refers to the DSC peak or melting point ±5°C or ±3°C. DSC provides an auxiliary method for distinguishing different crystal forms. Different crystal forms can be identified according to their different transition temperature characteristics. It should be pointed out that for mixtures, their DSC peaks or melting points may vary over a larger range. In addition, since decomposition is accompanied by the melting of the substance, the melting temperature is related to a heating rate.

For the same crystal form, the temperature of TGA weight loss may vary due to factors such as the measuring instrument, measuring method/conditions, etc. For any specific crystal form, the weight loss temperature may have an error of about ±5°C or about ±3°C.

It should be noted that when preparing drug crystal forms, during the contact between drug molecules and solvent molecules, it is difficult to avoid the situation that the solvent molecules and compound molecules form a co-crystal and remain in the solid material due to external conditions and internal factors, thereby forming a solvate, specifically including a stoichiometric solvate and a non-stoichiometric solvate. The solvates are all included in the scope of the present disclosure.

Unless otherwise specified, exothermic peaks are shown upward in a DSC plot. The therapeutic dose of the compounds of the disclosure may be determined, for example, based on the specific use of the treatment, the mode of administration of the compound, the health and state of the patient, and the judgment of the prescribing physician. The proportion or concentration of the compounds of the disclosure in the pharmaceutical composition may not be fixed, depending on a variety of factors, including dosage, chemical properties (e.g., hydrophobicity), and route of administration.

The term "treatment" means administering the compound or formulation of the present disclosure to improve or eliminate a disease or one or more symptoms associated with the disease, and includes:
(i) inhibiting a disease or disease state, i.e., arresting the development of the disease or disease state;
(ii) relieving a disease or disease state, i.e., regressing the disease or disease state.

The term "therapeutically effective amount" means the amount of a compound of the present disclosure that (i) treats a particular disease, condition, or disorder, (ii) alleviates, ameliorates, or eliminates one or more symptoms of a particular disease, condition, or disorder, or (iii) prevents or delays the onset of one or more symptoms of a particular disease, condition, or disorder described herein. The amount of a compound of the present disclosure that constitutes a "therapeutically effective amount" varies depending on the compound, the disease state and its severity, the mode of administration, and the age of the mammal to be treated, but can be routinely determined by those skilled in the art based on their own knowledge and this disclosure.

Unless otherwise required in the present disclosure, throughout the specification and the claims that follow, the word "comprise" and its English variations such as "comprises" and "comprising" should be interpreted as having an open and inclusive meaning, that is, "including but not limited to".

References to "one embodiment" or "an embodiment" or "in another embodiment" or "in certain embodiments" throughout this specification are intended to include in at least one embodiment the specific referenced elements, structures, or features described in connection with that embodiment. Thus, the phrases "in one embodiment" or "in an embodiment" or "in another embodiment" or "in certain embodiments" appearing in different places throughout the specification do not necessarily all refer to the same embodiment. Furthermore, the specific elements, structures, or features may be combined in any appropriate manner in one or more embodiments.

It should be understood that the singular article "a", "an" and "the" used in the present specification and the appended claims includes plural objects unless the context clearly dictates otherwise. Thus, for example, a reference to a reaction including "a catalyst" includes one catalyst, or two or more catalysts. It should also be understood that the term "or" is generally used in its sense including "and/or" unless the context clearly dictates otherwise.

The present disclosure will be described in detail below by way of examples, which are not intended to limit the present disclosure in any way.

All solvents used in the present disclosure were commercially available and used without further purification.

The compounds were named according to conventional nomenclature in the art or using ChemDraw^{®} software, and commercially available compounds were named according to the supplier's catalog name.

### X-ray powder diffractometer (XRPD) method of the present disclosure

Instrument model: Empyrean X-ray diffractometer
Test method: an appropriate amount of sample was evenly spreaded on a single crystal silicon sample plate and the XRPD test was performed using the following parameters.
The detailed XRPD parameters are as follows:
Instrument name: X-ray powder diffraction
Instrument manufacturer: Malvern Panalytical Co., Ltd.
Method parameters:
X-ray: Cu, kα, Kα1 (Å): 1.54060; Kα2 (Å): 1.54443; Kα2/Kα1 intensity ratio: 0.50
X-ray tube settings: 45 kV, 40 mA
Divergence slit: fixed 1/8°
Anti-scatter slit: 1/4°
Detector slit: P7.5
Scan mode: Continuous
Scanning range (°2Theta): 3-40
Scan time per step (s): 46.665
Scan step (°2Theta): 0.0263
Test time (min): 5

### Differential Scanning Calorimeter (DSC) method of the present disclosure

Instrument model: TA Discovery DSC 2500 Differential Scanning Calorimeter
Test method: the sample was placed in an alumina crucible for testing, and the sample was heated from RT to 320°C at a heating rate of 10°C/min.

### Thermogravimetric analysis (TGA) method of the present disclosure

Instrument model: TA Discovery TGA 5500 Thermogravimetric Analyzer and Thermogravimetric Analyzer
Test method: The sample was placed in a DSC high pressure crucible, pressed and sealed for testing. The sample was heated from 25°C to 350°C at a heating rate of 10°C/min.

### BRIEF DESCRIPTION OF THE DRAWINGS

Fig. 1 is an XRPD spectrum of Cu-Kα radiation of crystal form A of the compound of formula (I);
Fig. 2 is a DSC spectrum of crystal form A of the compound of formula (I);
Fig. 3 is a TGA spectrum of crystal form A of the compound of formula (I).

### DETAILED DESCRIPTION

The present disclosure is described in detail below by way of examples, but it is not intended to limit the present disclosure in any way. The present disclosure has been described in detail herein, and specific embodiments thereof are also disclosed therein. It will be apparent to those skilled in the art that various changes and modifications can be made to the specific embodiments of the present disclosure without departing from the spirit and scope of the present disclosure.

### Example 1: Preparation of the compound of formula (I)

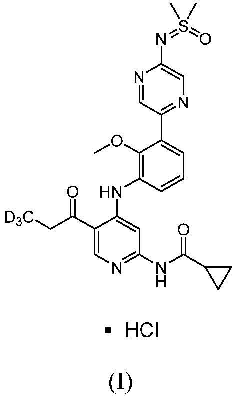

### Synthesis route:

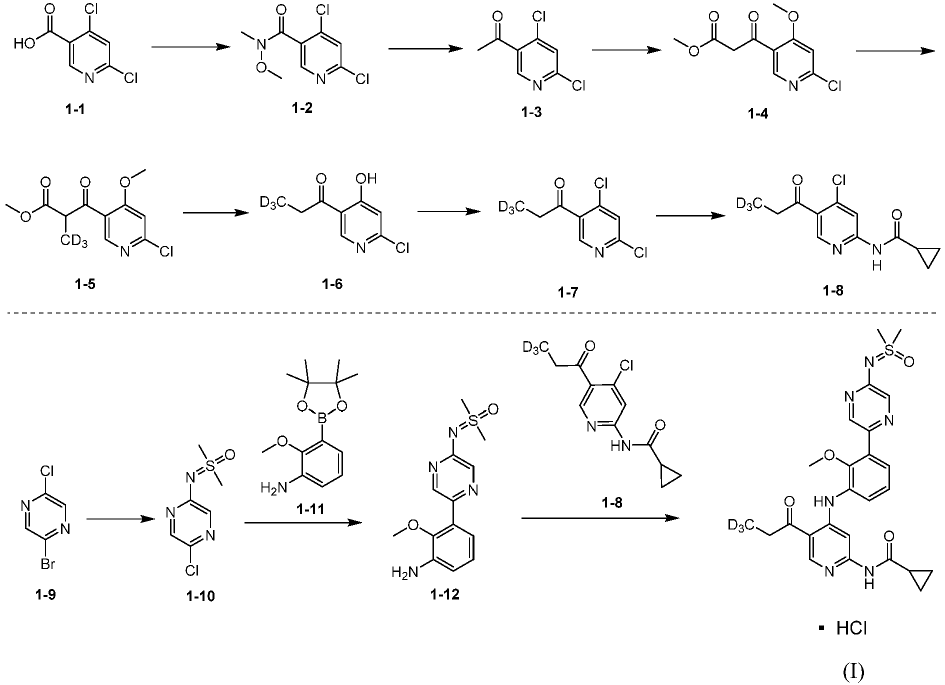

### Step 1: Synthesis of compound 1-2

To a solution of compound **1-1** (13 g, 67.71 mmol) in dichloromethane (200 mL) were added N,N-diisopropylethylamine (43.75 g, 338.54 mmol) and O-(7-azabenzotriazol-1-yl)-N,N,N,N-tetramethyluronium hexafluorophosphonate (30.89 g, 81.25 mmol). The mixture was stirred at 20 °C for 0.5 hours, and then N,O-dimethylhydroxylamine hydrochloride (7.93 g, 81.25 mmol) was added. The mixture was stirred at 20 °C for 15.5 hours. The reaction solution was concentrated, and the resulting crude product was purified by column chromatography (silica gel, petroleum ether/ethyl acetate = 10/1 ~ 3/1) to obtain compound **1-2.**

MS *m*/*z:* 235 [M+H]⁺.

### Step 2: Synthesis of Compound 1-3

Under nitrogen protection at 0 °C, to a solution of compound **1-2** (13 g, 55.30 mmol) in tetrahydrofuran (130 mL) was added methylmagnesium bromide (3M, solution in diethyl ether, 36.87 mL). The mixture was stirred at 0 °C for 2 hours. The reaction solution was quenched with saturated aqueous ammonium chloride solution (60 mL), diluted with water (100 mL), and then extracted with ethyl acetate (100 mL×2). The organic phases were combined and washed with saturated brine (100 mL×2), dried over anhydrous sodium sulfate, and filtered. The filtrate was concentrated under reduced pressure to give a crude product, which was purified by column chromatography (silica gel, petroleum ether/ethyl acetate = 20/1 ~ 15/1) to obtain compound **1-3.**

¹H NMR (400 MHz, CDCl₃) δ 8.59 (s, 1H), 7.44 (s, 1H), 2.66 (s, 3H).

### Step 3: Synthesis of Compound 1-4

To a solution of **1-3** (8 g, 42.10 mmol) and dimethyl carbonate (42.80 g, 475.15 mmol) in tetrahydrofuran (40 mL) was slowly added 60% sodium hydride (5.05 g, 126.30 mmol) in batches at 0 °C, and the mixture was stirred at 20 °C for 16 hours. The reaction solution was diluted with ethyl acetate (100 mL), quenched with aqueous hydrochloric acid (50 mL 2M), and washed with saturated brine (50 mL × 2). The organic phase was dried over anhydrous sodium sulfate, and filtered. The filtrate was concentrated under reduced pressure to give a crude product. The crude product was purified by column chromatography (silica gel, petroleum ether/ethyl acetate = 10/1 ~ 3/1) to give compound **1-4.**

MS *m*/*z:* 244 [M+H]⁺.

### Step 4: Synthesis of Compound 1-5

To a solution of compound **1-4** (8.2 g, 32.65 mmol) and potassium carbonate (4.96 g, 35.91 mmol) in N,N-dimethylformamide (80 mL) was added deuterated iodomethane (4.97 g, 34.28 mmol) at 0 °C, and the mixture was stirred at 20 °C for 6 hours. The reaction solution was diluted with water (100 mL), and extracted with ethyl acetate (100 mL×2). The organic phases were combined, washed with saturated brine (100 mL×2), dried over anhydrous sodium sulfate, and filtered. The filtrate was concentrated under reduced pressure to give a crude product. The product was purified by column chromatography (silica gel, petroleum ether/ethyl acetate = 20/1 ~ 4/1) to give compound **1-5.**

MS *m*/*z:* 261 [M+H]⁺.

### Step 5: Synthesis of Compound 1-6

To a solution of **1-5** (5.8 g, 18.02 mmol, 81%) in acetic acid (30 mL) was added 35% concentrated hydrochloric acid (61.20 g, 587.48 mmol), and the mixture was stirred at 130 °C for 16 hours. The reaction solution was concentrated under reduced pressure to give a crude product, which was purified by column chromatography (silica gel, petroleum ether/ethyl acetate = 30/1 ~ 1/1) to give compound **1-6.**

MS *m*/*z:* 189 [M+H]⁺.

### Step 6: Synthesis of Compound 1-7

To a solution of **1-6** (2.5 g, 12.33 mmol) in acetonitrile (80 mL) was added phosphorus oxychloride (7.56 g, 49.30 mmol), and the mixture was stirred at 85 °C for 1 hour. The reaction solution was concentrated and diluted with ethyl acetate (100 mL). The organic phase was washed with saturated aqueous sodium bicarbonate solution (50 mL × 3), dried over anhydrous sodium sulfate, and filtered. The filtrate was concentrated under reduced pressure to give a crude product, which was purified by column chromatography (silica gel, petroleum ether/ethyl acetate = 1/0 ~ 50/1) to give compound **1-7.**

MS *m*/*z:* 207 [M+H]⁺.

### Step 7: Synthesis of Compound 1-8

A solution of compound **1-7** (50 mg, 241.46 µmol), cyclopropylcarboxamide (20.55 mg, 241.46 µmol), potassium carbonate (66.75 mg, 482.92 µmol), 4,5-bis(diphenylphosphino)-9,9-dimethylxanthene (27.94 mg, 48.29 µmol) and tris(dibenzylideneacetone)dipalladium chloroform complex (22.11 mg, 24.15 µmol) in dioxane (2 mL) was purged three times with nitrogen. The mixture was stirred at 80 °C for 2 hours. The reaction solution was concentrated to give a crude product, which was purified by column chromatography (silica gel, petroleum ether/ethyl acetate = 20/1 ~ 10/1) to give compound **1-8.**

MS *m*/*z:* 256 [M+H]⁺.

### Step 8: Synthesis of Compound 1-10

Compound **1-9** (2 g, 10.34 mmol) was dissolved in dioxane (40 mL). Dimethylsulfoximine (1.01 g, 10.86 mmol), cesium carbonate (6.74 g, 20.68 mmol), tris(dibenzylideneacetone)dipalladium (946.85 mg, 1.03 mmol) and 4,5-bis(diphenylphosphino)-9,9-dimethylxanthene (1.20 g, 2.07 mmol) were added. The atmosphere was replaced with nitrogen three times, and the mixture was heated to 110 °C and stirred under nitrogen for 4 hours. The reaction solution was directly concentrated under reduced pressure to give a crude product, which was purified by column chromatography (silica gel, petroleum ether/ethyl acetate = 10/1 ~ 1/4). The fraction was concentrated under reduced pressure, and stirred with petroleum ether/ethyl acetate = 5/1 (12 mL) at 20 °C for 1 hour. The mixture was filtered, and the filter cake was collected and dried to give compound **1-10.**

MS *m*/*z:* 206 [M+H]⁺.

### Step 9: Synthesis of Compound 1-12

Compound **1-10** (400 mg, 1.94 mmol) was dissolved in dioxane (8 mL) and water (2 mL). Compound **1-11** (532.95 mg, 2.14 mmol), potassium phosphate (825.68 mg, 3.89 mmol), and 1,1-bis(diphenylphosphino)ferrocene palladium chloride (142.31 mg, 194.49 µmol) were added. The atmosphere was replaced with nitrogen three times, and the mixture was heated to 100 °C, and stirred under nitrogen for 2 hours. The reaction solution was directly concentrated under reduced pressure to give a crude product, which was purified by column chromatography (silica gel, petroleum ether/ethyl acetate = 5/1 ~ 0/1). The fraction was concentrated under reduced pressure, and stirred with petroleum ether/ethyl acetate = 1/1 (4 mL) at 20 °C for 1 hour. The mixture was filtered, and the filter cake was collected and dried to give compound **1-12.**

MS *m*/*z:* 293 [M+H]⁺.

### Step 10: Synthesis of compound of formula (I)

Compound **1-12** (635 g) and compound **1-8** (666.5 g) were dissolved in isopropanol (3.175 L). Concentrated hydrochloric acid (22.63 mL) was subsequently added. The internal temperature was 65-70°C and the reaction was carried out for 16-20 hours. After the reaction was completed, the mixture was filtered while hot, and the filter cake was washed with isopropanol (5 L). The filter cake was collected to obtain a compound of formula (I).

HNMR: ¹H NMR (400 MHz, DMSO-d6) δ ppm 11.91 - 12.23 (m, 1 H) 11.25 (br d, J=4.77 Hz, 1 H) 8.89 (d, J=2.01 Hz, 1 H) 8.61 (s, 1 H) 8.15 (d, J=1.51 Hz, 1 H) 7.67 (br d, J=7.78 Hz, 1 H) 7.15 - 7.53 (m, 3 H) 3.33 - 3.60 (m, 9 H) 3.11 (s, 2 H) 1.94 (br d, J=4.77 Hz, 1 H) 0.77 - 1.00 (m, 4 H).

### Example 2: Preparation of crystal form A of the Compound of Formula (I)

The compound of formula (I) (126 g) was weighed and added to methanol (2900 mL). The reaction solution was stirred at 55°C for 1.5 hours, and mercapto silica gel (63 g) was added. The mixture was stirred at 55°C for 17.5 hours, and filtered under reduced pressure. The filtrate was concentrated to 2200-2400 mL at ≤50°C, and 250 mL of tert-butyl methyl ether was added. The mixture was stirred at 55°C for 1.5 hours. Seed crystals were added and 2500 mL of tert-butyl methyl ether was added dropwise at 55°C. The dropwise addition continued for more than 8 hours. The mixture was slowly cooled to -20-0°C, and the cooling time was more than 6 hours. The mixture was stirred at this temperature for 42 hours. The reaction mixture was filtered with suction under reduced pressure. The filter cake was washed with 400 mL of tert-butyl methyl ether. The filter cake was dried at 50-60°C under vacuum (-0.08-0.1 MPa) for 23 hours to obtain the crystal form A of the compound of formula (I).

### Example 3: Solid Stability Test of crystal form A of Compound of Formula (I)

About 1.5 g of crystal form A of the compound of formula (I) was respectively weighed, placed at the bottom of a glass sample bottle, and spread into a thin layer. The sample was completely exposed. The samples that were placed at high temperature and high humidity were sampled and tested on the 10th day and 30th day (XRPD). For illumination, the samples were placed in a clean surface dish, spread into a thin layer, and covered with a quartz glass cover, and then sampled and tested on the 5th day and 10th day (XRPD). For long-term tests and accelerated tests (dark), each sample was placed in a double-layer LDPE bag, and each layer of the LDPE bag was sealed with buckles. Then the LDPE bag was placed in an aluminum foil bag that has been added with a bag of desiccant. The aluminum foil bag was heat-sealed, and placed under 25°C/60%RH, 30°C/65%RH and 40°C/75%RH conditions for inspection. The test results were compared with the initial test results of Day 0. The test results are shown in Table 2 below:

**Table 2: Results of the solid stability test of crystal form A of the compound of formula (I)**

| Test conditions | Time | Crystal form |
|---|---|---|
| - | Day 0 | Crystal form A |
| High temperature (60°C) | Day 10 | Crystal form A |
| | Day 30 | Crystal form A |
| High humidity (25°C/relative humidity 92.5%) | Day 10 | Crystal form A |
| | Day 30 | Crystal form A |
| Strong illumination (5000 lx and UV intensity 90w/cm², open) | Day 5 | Crystal form A |
| | Day 10 | Crystal form A |
| 25°C/60%RH | 3M | Crystal form A |
| | 6M | Crystal form A |
| 30°C/65%RH | 1M | Crystal form A |
| | 3M | Crystal form A |
| | 6M | Crystal form A |
| 40°C/75%RH | 1M | Crystal form A |
| | 3M | Crystal form A |
| | 6M | Crystal form A |

**Conclusion:** Crystal form A of the compound of formula (I) has good stability under the conditions of high temperature, high humidity, strong illumination, and long-term accelerated test conditions.

### Biological evaluation

### Experimental Example 1: In vitro enzyme activity evaluation

### Experimental process of Tyk2 JH2 enzyme activity test

To a buffer solution containing 20 mM of Hepes pH 7.5, 10 mM of MgCl₂, 0.015% of Brij-35, 2 mM of DTT and 50 µg/ mL of BSA were added 0.5 nM of TYK2 protein (His-TVMV-TYK2 JH2 (575-869)), 0.2 nM of terbium-labeled His antibody, fluorescein-labeled kinase tracer at the relevant K_{d} value, and the test compound. The test system was incubated at room temperature for 90 minutes. Subsequently, the generated HTRF (homogeneous time-resolved fluorescence) signal, i.e., the ratio of the fluorescence intensity of the fluorescein acceptor (520 nm) and the terbium donor (495 nm) at the emission wavelength, was measured on an Envision plate reader, and the IC50 value was calculated based on this. The results of in vitro enzyme activity assay of the free base of the compound of the present disclosure are shown in Table 3:

**Table 3: In vitro enzyme activity test results of the compound of the present disclosure (IC₅₀)**

| No. | Tyk2 JH2 (IC₅₀ nM) |
|---|---|
| The free base of the compound of formula (I) | 0.04 |

Experimental conclusion: The free base of the compound of the present disclosure has a strong inhibitory activity against Tyk2 JH2.

### Experimental Example 2: In vitro cell activity evaluation

### IFNα-stimulated phosphorylation STAT1 assay

Human peripheral blood mononuclear cells (hPBMC) were seeded at a cell density of 1x10⁵ cells/well and placed in an incubator at 37°C for 90 minutes. Then different concentrations of compounds were added to the cells, wherein the concentration range of each compound started from 2 µM, which was serially diluted 5-fold to obtain a total of 8 concentration gradients. The compounds and cells were incubated at 37 °C for 30 minutes. hPBMC were stimulated with IFN-a (1000 U/ml), and then the level of phosphorylated STAT1 in CD4+ T cells was detected by flow cytometry to evaluate the inhibitory activity of the compound on the IFN-α pathway. The results of the in vitro cell activity assay of the free base of the compound of the present disclosure are shown in Table 4:

**Table 4: In vitro cell activity test results of the compound of the present disclosure (IC₅₀)**

| No. | IFNα-stimulated phosphorylated STAT1 (IC₅₀ nM) |
|---|---|
| The free base of the compound of formula (I) | 1.1 |

Experimental conclusion: The free base of the compound of the present disclosure has a strong Tyk2-related cellular activity on IFNα-stimulated phosphorylated STAT1.

### Experimental Example 3: Pharmacokinetic Study (PK)

### Experimental purpose:

The purpose of this experiment is to study the pharmacokinetics of the test substance in the plasma of male CD-1 mice, SD rats and pit bulls after intravenous injection and oral administration.

### Experimental methods:

The animals were randomly divided into two groups, with 2 males in each group. The compounds were formulated into the specified formulations (the vehicle was 5 mg/mL of 10% sulfobutyl ether-β-cyclodextrin), and the oral formulations were clear or homogeneous suspensions.

Whole blood samples were collected from the animals by jugular vein puncture or saphenous vein at 5, 15, 30 minutes, 1, 2, 4, 8, and 24 hours after administration. The whole blood samples were added into centrifuge tubes containing anticoagulants, and centrifuged at 3000g for 15 minutes at 4°C. The supernatant plasma was collected and quickly frozen on dry ice and then stored in a refrigerator at -70 ± 10°C until LC-MS/MS analysis.

### Data processing:

The pharmacokinetic software WinNonlin^{™} Version 6.3.0 (Pharsight, Mountain View, CA) was used to process the plasma drug concentration data of the compound in a non-compartmental model. The peak concentration (Cₘₐₓ) and time to peak (Tₘₐₓ) as well as last measurable time point were directly obtained from the plasma drug concentration-time graph.

The following pharmacokinetic parameters were calculated using the log-linear trapezoidal method: plasma clearance rate (CL), volume of distribution (Vd), elimination phase half-life (T_{1/2}), mean residence time of drug in vivo from 0 to the terminal time point (MRT₀₋ₗₐₛₜ), mean residence time of drug in vivo from 0 to infinite time (MRT_{0-inf}), area under the time-plasma concentration curve from 0 to the terminal time point (AUC₀₋ₗₐₛₜ), area under the time-plasma concentration curve from 0 to infinite time (AUC_{0-inf}), and bioavailability (F). The experimental results of the present disclosure are shown in Table 5:

**Table 5: Pharmacokinetic test results**

| | | | Mouse | Rat | Dog |
|---|---|---|---|---|---|
| | | | **Compound of formula (I)** | | |
| **PK** | **PO** | **Dose (mpk)** | **10** | **30** | **25** |
| | | **Cmax (nM)** | 32400 | 44450 | 16789 |
| | | **Tmax (h)** | 0.5 | 0.75 | 2.0 |
| | | **AUC_{0~last}(nM.h)** | 76037 | 231209 | 66504 |
| | | **F (%)** | 81 | 65 | 103 |

| | | | | | |
|---|---|---|---|---|---|
| Note: Vd: volume of distribution; Cl: clearance rate; T_{1/2}: half-life; AUC: exposure (area under the curve); Cₘₐₓ: maximum concentration; Tₘₐₓ: time to peak concentration; F%: bioavailability; PO: oral administration | | | | | |

Experimental conclusion: The compound of the present disclosure shows excellent pharmacokinetic properties, and high in vivo exposure and oral bioavailability.

## Claims

1. A compound of formula (I),

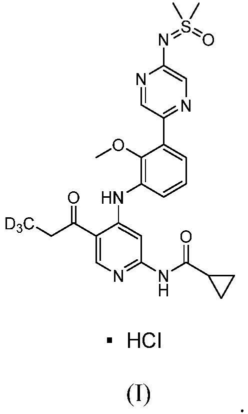

2. Crystal form A of the compound of formula (I) according to claim 1, wherein the X-ray powder diffraction pattern thereof has characteristic diffraction peaks at the following 2θ angles: 17.56±0.20°, 24.36±0.20°, 25.69±0.20°, and 27.37±0.20°.

3. Crystal form A of the compound of formula (I) according to claim 1, wherein the X-ray powder diffraction pattern of the crystal form A at least contains 6, 7 or 8 diffraction peaks expressed by 2θ angle selected from: 17.56±0.20°, 18.76±0.20°, 19.63±0.20°, 20.44±0.20°, 22.89±0.20°, 24.36±0.20°, 25.69±0.20°, and 27.37±0.20°.

4. Crystal form A of the compound of formula (I) according to claim 1, wherein the X-ray powder diffraction pattern thereof has characteristic diffraction peaks at the following 2θ angles: 17.56±0.20°, 18.76±0.20°, 19.63±0.20°, 20.44±0.20°, 22.89±0.20°, 24.36±0.20°, 25.69±0.20°, and 27.37±0.20°.

5. Crystal form A of the compound of formula (I) according to claim 1, wherein the X-ray powder diffraction pattern of the crystal form A at least contains 10, 11 or 12 diffraction peaks expressed by 2θ angle selected from: 8.70±0.20°, 10.05±0.20°, 15.56±0.20°, 17.56±0.20°, 18.76±0.20°, 19.63±0.20°, 20.44±0.20°, 22.89±0.20°, 24.36±0.20°, 25.69±0.20°, 27.37±0.20°, and 29.49±0.20°.

6. Crystal form A of the compound of formula (I) according to claim 1, wherein the X-ray powder diffraction pattern thereof has characteristic diffraction peaks at the following 2θ angles: 8.70±0.20°, 10.05±0.20°, 15.56±0.20°, 17.56±0.20°, 18.76±0.20°, 19.63±0.20°, 20.44±0.20°, 22.89±0.20°, 24.36±0.20°, 25.69±0.20°, 27.37±0.20°, and 29.49±0.20°.

7. Crystal form A of the compound of formula (I) according to claim 1, wherein the X-ray powder diffraction pattern of the crystal form A at least contains 12, 13, 14, 15 or 16 diffraction peaks expressed by 2θ angle selected from: 8.70±0.20°, 10.05±0.20°, 12.08±0.20°, 15.56±0.20°, 17.56±0. 20°, 18.76±0.20°, 19.63±0.20°, 20.44±0.20°, 22.89±0.20°, 23.77±0.20°, 24.36±0.20°, 25.69±0.20°, 26.33±0.20°, 27.37±0.20°, 27.83±0.20°, and 29.49±0.20°.

8. Crystal form A of the compound of formula (I) according to claim 1, wherein the X-ray powder diffraction pattern thereof has characteristic diffraction peaks at the following 2θ angles: 8.70±0.20°, 10.05±0.20°, 12.08±0.20°, 15.56±0.20°, 17.56±0.20°, 18.76±0.20°, 19.63±0.20°, 20.44±0.20°, 22.89±0.20°, 23.77±0.20°, 24.36±0.20°, 25.69±0.20°, 26.33±0.20°, 27.37±0.20°, 27.83±0.20°, and 29.49±0.20°.

9. Crystal form A of the compound of formula (I) according to claim 1, wherein the X-ray powder diffraction peak spectrum thereof has characteristic diffraction peaks at the following 2θ angles: 17.56°±0.20°, 24.36°±0.20°, 27.37°±0.20°, and/or 8.70°±0.20°, and/or 10.05°±0.20°, and/or 12.08°±0.20°, and/or 14.63°±0.20°, and/or 15.56°±0.20°, and/or 16.39°±0.20°, and/or 18.76°±0.20°, and/or 19.35°±0.20°, and/or 19.63°±0.20°, and/or 20.44°±0.20°, and/or 21.94°±0.20°, and/or 22.53°±0.20°, and/or 22.89°±0.20°, and/or 23.23°±0.20°, and/or 23.77°±0.20°, and/or 24.51°±0.20°, and/or 25.69°±0.20°, and/or 26.33°±0.20°, and/or 26.73°±0.20°, and/or 27.83°±0.20°, and/or 28.58°±0.20°, and/or 29.49°±0.20°, and/or 30.42°±0.20°, and/or 31.13°±0.20°, and/or 32.29°±0.20°, and/or 32.88°±0.20°, and/or 34.04°±0.20°, and/or 35.19°±0.20°, and/or 36.30°±0.20°, and/or 38.89°±0.20°.

10. Crystal form A of the compound of formula (I), 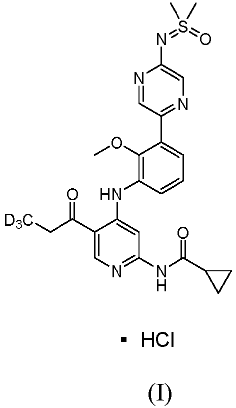 wherein the XRPD pattern is shown in Fig. 1.

11. Crystal form A of the compound of formula (I) according to any one of claims 1 to 10, wherein the differential scanning calorimetry curve thereof has a starting point of an endothermic peak at 230.5°C±3.0°C.

12. The crystal form A of the compound of formula (I) according to claim 11, wherein the DSC spectrum thereof is shown in Fig. 2.

13. Crystal form A of the compound of formula (I) according to any one of claims 1 to 10, wherein the thermogravimetric analysis curve thereof shows a weight loss of up to 1.02%±0.20% at 175.0°C±3.0°C.

14. The crystal form A of the compound of formula (I) according to claim 13, wherein the TGA spectrum thereof is shown in Fig. 3.

15. Use of the compound according to claim 1 or the crystal form A of the compound of formula (I) according to any one of claims 2 to 14 in the manufacture of a medicament for treating Tyk2 JH2 related.
